# EUROPEAN PATENT SPECIFICATION

(11) **EP 4 146 094 B1**
(45) Date of publication and mention of the grant of the patent: **17.12.2025**
(21) Application number: 20807053.2
(22) Date of filing: 19.11.2020
(51) Int. Cl.: A61B 17/15

(54) **4-IN-1 CUTTING JIG FOR FEMORAL FINISHING**
4-IN-1 SCHNEIDVORRICHTUNG ZUR ENDBEARBEITUNG DES FEMURS
GABARIT DE COUPE 4-EN-1 POUR FINITION FÉMORALE

(30) Priority: 08.05.2020 IT 202000010393
(43) Date of publication of application: 15.03.2023
(73) Proprietor: REJOINT S.r.l., 40127 Bologna (IT)
(72) Inventor: RIVA, Gian-Guido, 40123 Bologna (IT)
(74) Representative: Rapisardi, Mariacristina
(86) International application number: PCT/EP2020/082709
(87) International publication number: WO 2021/223895

(56) References cited:
- EP-A1- 2 173 260
- WO-A1-2016/138913
- WO-A1-96/25114

## Description

The present patent application relates to a 4-in-1 cutting jig for femoral finishing. In particular, the present invention relates to a made-to-measure 4-in-1 cutting jig adapted for the execution of an anterior resection, a posterior resection, an anterior chamfer and a posterior chamfer of the femur.

Performing a distal resection on the femur of a patient and subsequently a femoral finishing that comprises the execution of an anterior resection, a posterior resection, an anterior chamfer and a posterior chamfer is known. During the execution of the femoral finishing, the correct execution of the resections and chamfers is decisive for the correct positioning of the prosthesis.

For the execution of femoral finishing, the use of reusable multi-use metal instruments, whose correct positioning is substantially left up to the abilities of the surgeon at the time of the operation, is known.

In practice, a 4-in-1 cutting jig is provided to guide the cutting blades for the execution of the anterior and posterior resections, and the anterior and posterior chamfers.

However, in order to be able to obtain a desired internal or external rotation of the knee the surgeon has to manually adjust the inclination of the 4-in-1 cutting jig.

Therefore, the known 4-in-1 cutting jig of the above-described type has the disadvantage of not ensuring the correct positioning for obtaining a given internal or external rotation, with consequent problems, in the event of error, in the positioning of the prosthesis and in the articulation of the knee.

Patient-specific single-use 4-in-1 cutting jigs are also known; they are generally made with additive technology, in which the morphology of the area of intervention of the femur is preliminarily acquired instrumentally, and on the basis of the reconstruction obtained a 3D printer is guided for the production of a customised cutting jig.

This solution is functional but rather costly, since every piece produced can obviously be used for only one patient, and is imprecise in guiding the cutting blades.

<EP 2 173 260 A1 discloses a cutting jig having a cutting block and an adjustable module. WO 2016/138913 Al discloses a custom made cutting jig.>

The object of the present invention is to provide a 4-in-1 cutting jig for femoral finishing that overcomes the above-mentioned problems.

The object of the present invention is to provide a 4-in-1 cutting jig for femoral finishing that is patient specific, but economical.

This and other objects of the invention are achieved by a 4-in-1 cutting jig for femoral finishing comprising a cutting block provided with cutting guides configured to be placed, during use, in contact with the distal resection surface of a femur, wherein it comprises at least a first patient-specific made-to-measure module and first coupling means in an obligatory position for coupling said first module with said cutting block, said first module having first reference means configured to position, during use, said cutting block in a predetermined patient-specific manner relative to said distal resection surface, said first reference means comprising at least a first abutment element having a first surface configured to line up, during use, with a femoral landmark of the patient.

The cutting jig according to the invention has an extremely high flexibility of use, since the cutting block is universal whilst the first module, which is replaceable, renders the cutting jig patient specific.

In practice, for the use thereof, the universal cutting block is employed and a module specifically made for the patient is coupled to the universal cutting block. In this manner, though the main component of the cutting jig is reusable for different patients, each patient can have his or her own customised cutting jig which ensures the correct execution of the surgical intervention.

In one embodiment of the invention said first reference means consist of a first and respectively a second abutment element having a first and respectively a second surface configured to line up, during use, with the lateral femoral and respectively medial femoral condyle of the patient.

In one embodiment of the invention, said first reference means consist of a single abutment element having a wedge-shaped surface configured to line up, during use, with the femoral intracondyloid fossa of the patient.

In one embodiment of the invention, said first coupling means in an obligatory position comprise geometrically conjugate surfaces of said first module and said cutting block.

In one embodiment of the invention, said first coupling means in an obligatory position comprise at least one locking screw for locking between said geometrically conjugate surfaces of said first module and said cutting block.

In one embodiment of the invention, there is provided at least a second made-to-measure patient-specific module and second coupling means in an obligatory position for coupling said second module with said cutting block, said second module having second reference means configured to position, during use, said cutting block in a predetermined patient-specific manner relative to said distal resection surface, said second reference means comprising a profile of said second module configured to be aligned, during use, with anterior anatomical landmarks of the femur adjacent to the distal resection surface.

In one embodiment of the invention, said second coupling means in an obligatory position comprise quick connect elements for connecting between said second module and said cutting block.

In one embodiment of the invention, said cutting block has, distributed along a first axis parallel to the anteroposterior axis of the femur:
- a front guide having a first guide surface configured to guide, during use, a cutting instrument during the execution of an anterior resection;
- a front guide having a second guide surface configured to guide, during use, a cutting instrument during the execution of an anterior chamfer;
- a rear guide having a third guide surface configured to guide, during use, a cutting instrument during the execution of a posterior chamfer;
- a rear guide having a fourth guide surface configured to guide, during use, a cutting instrument during the execution of a posterior resection.

In one embodiment of the invention, said cutting block and said first module are made of a different material.

In one embodiment of the invention, said first module is produced with additive technology.

The invention will now be described with reference to the accompanying drawings, which illustrate non-limiting example embodiments, wherein:
- figures 1 - 3 show exploded perspective and plan views of the cutting jig with abutment elements on the condyles;
- figures 4 - 7 show perspective and plan views of the cutting jig with abutment elements on the condyles, wherein the jig is assembled and mounted on the distal resection surface of the femur;

- figures 8 - 11 show views, in an orthogonal projection, of the cutting jig with abutment elements on the condyles, wherein the jig is assembled and mounted on the distal resection surface of the femur;

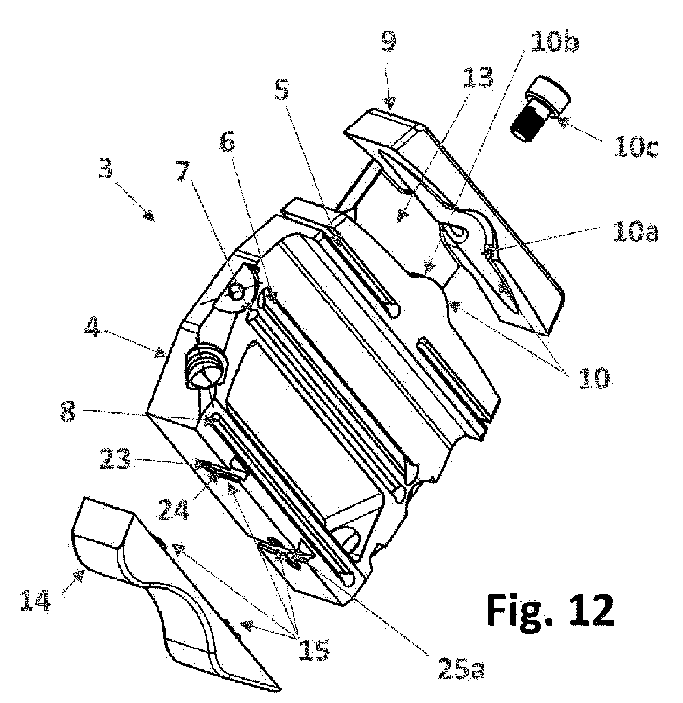

- figure 12 shows an exploded perspective view of the cutting jig with the single abutment element in the intracondyloid fossa of the femur;

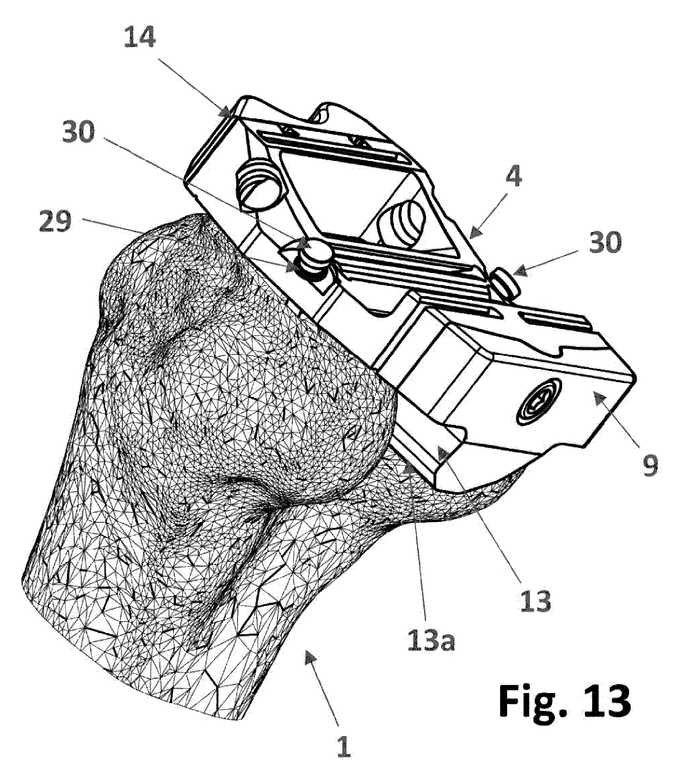

- figure 13 shows a perspective view of the cutting jig with the single abutment element in the intracondyloid fossa of the femur, wherein the jig is assembled and mounted on the distal resection surface of the femur.

Equivalent elements in the various embodiments of the cutting jig will be indicated with the same numerical reference.

The terms representing anatomical references such as distal, proximal, anterior, posterior, medial, lateral, superior and inferior are used hereinbelow with reference to the anatomy of a patient, front and rear with reference to the 4-in-1 cutting jig 3 described and to orthopaedic prostheses.

The figures illustrate a femur 1, in particular a distal resection surface 2 obtained following a distal resection of the femur 1 itself, and a 4-in-1 cutting jig 3 for femoral finishing.

The cutting jig 3 is a surgical instrument that is used in the operating phase to finish the femur following a distal resection and facilitate the implantation of a prosthesis.

The jig 3 is configured to be able to guide cutting instruments during the execution of four different cuts, in particular to be able to execute: an anterior resection, a posterior resection, an anterior chamfer and a posterior chamfer.

In particular, the cutting jig 3 is applied on the patient only during the operation, but is removed for the implantation of a prosthesis.

The cutting jig 3 comprises a cutting block 4 provided with cutting guides 5, 6, 7, and 8.

The cutting block 4 is configured to be placed, during use, in contact with the distal resection surface of the femur 1.

The cutting jig 3 advantageously comprises at least a first made-to-measure patient-specific module 9 and first coupling means 10 in an obligatory position for coupling the first module 9 with the cutting block 4.

The term "patient-specific made-to-measure" means that the first module 9 is configured to be used, during an orthopaedic operation, on a specific patient.

The first module 9 has first reference means configured to position, during use, the cutting block 4 in a predetermined patient-specific manner relative to the distal resection surface 2.

The first reference means comprising at least a first abutment element 11, 12, 13 having a first surface configured to line up, during use, with a femoral landmark of the patient.

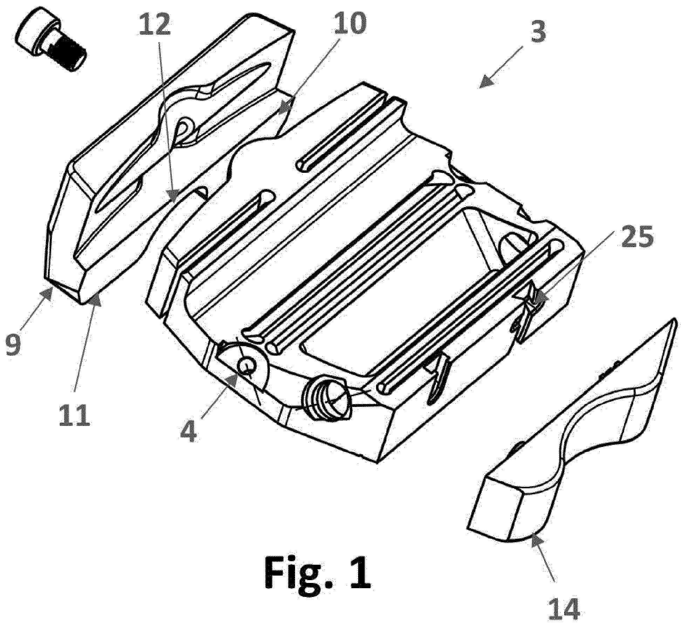

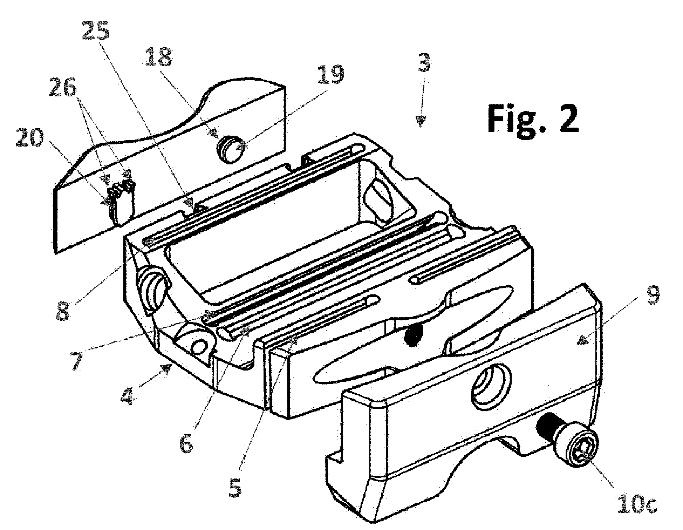

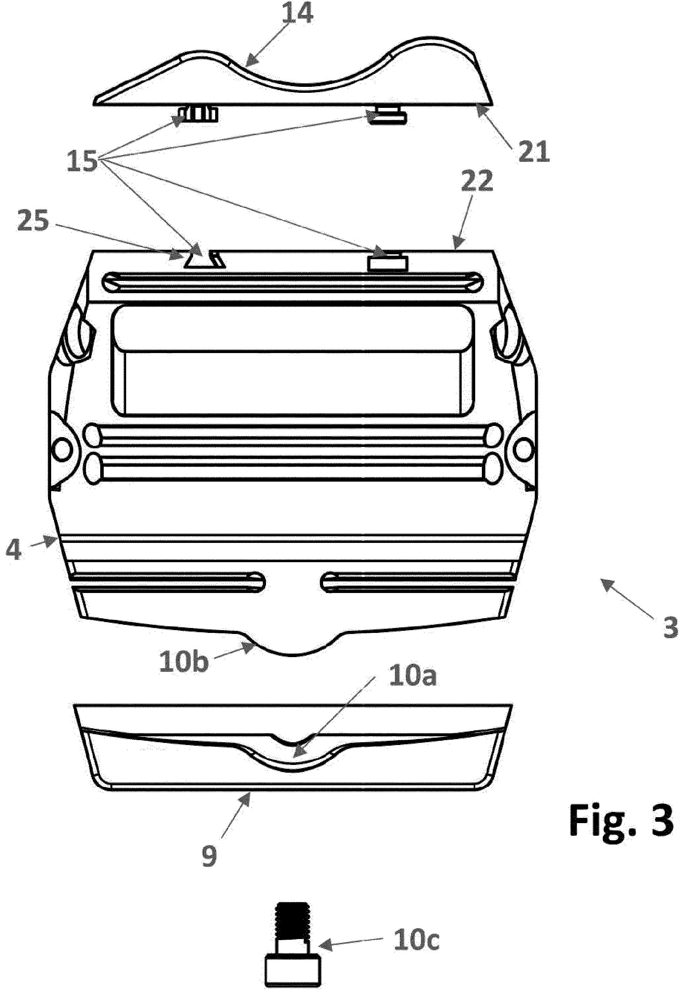

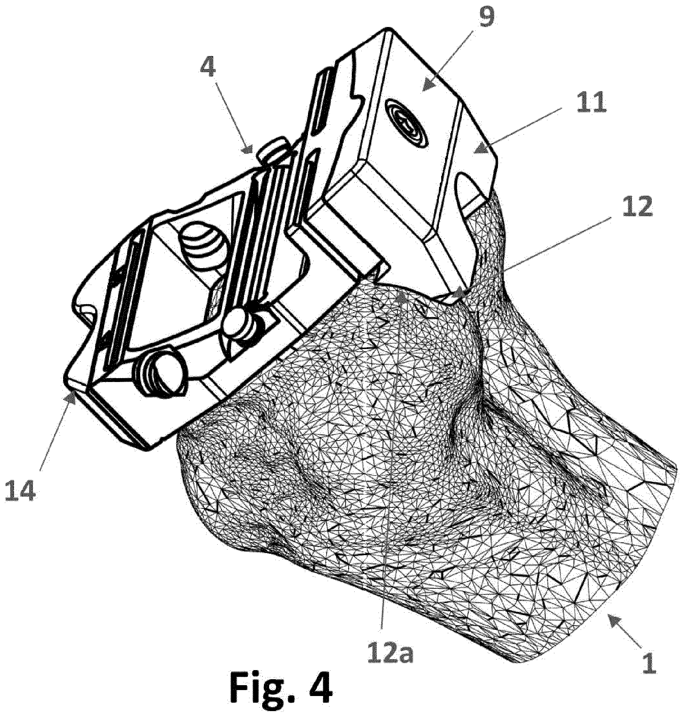

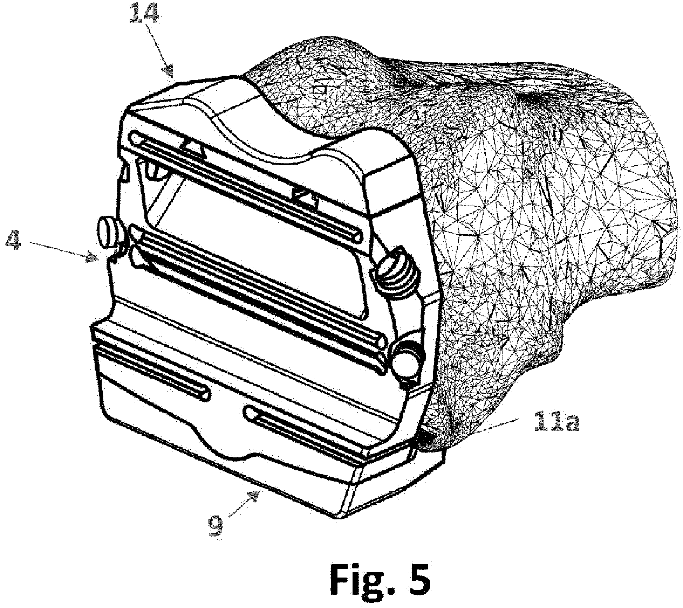

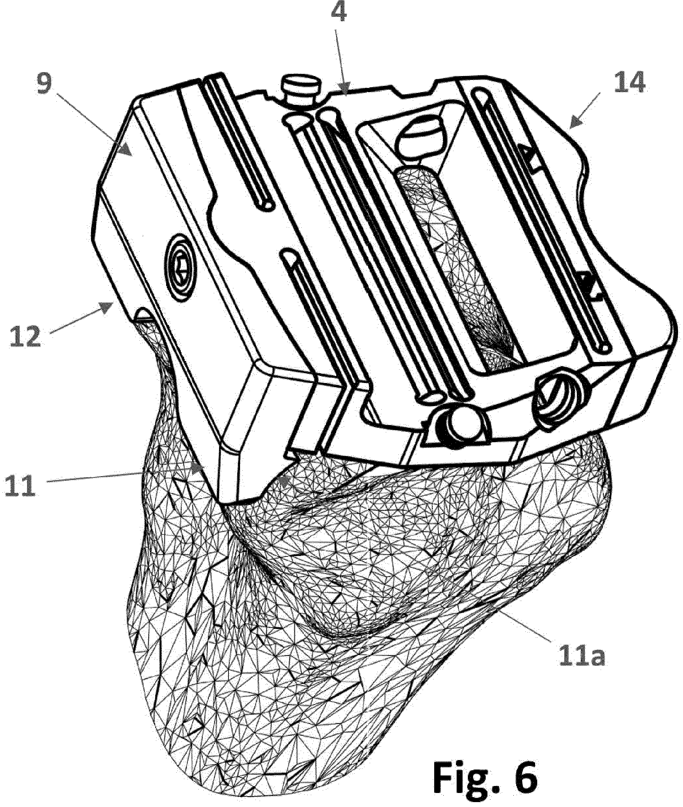

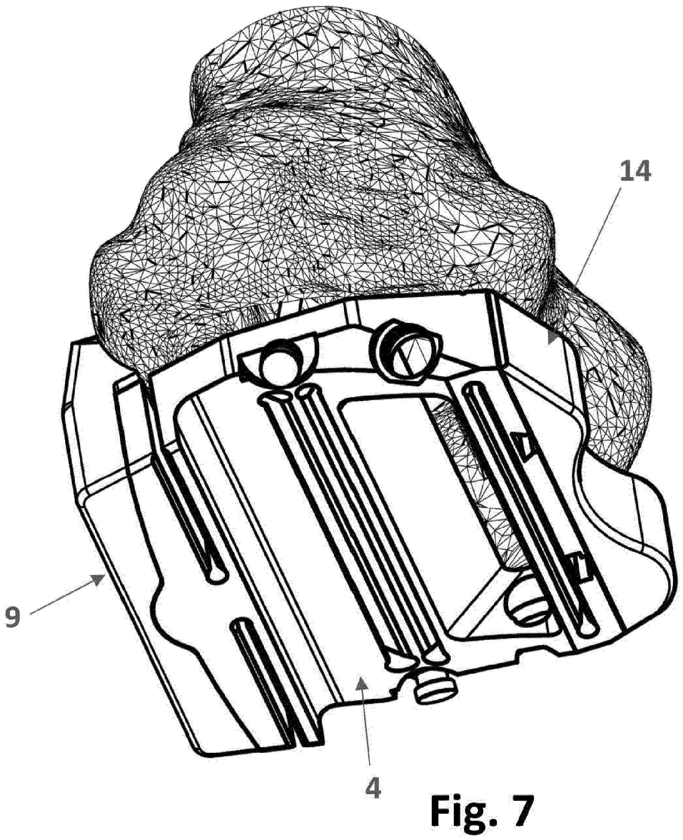

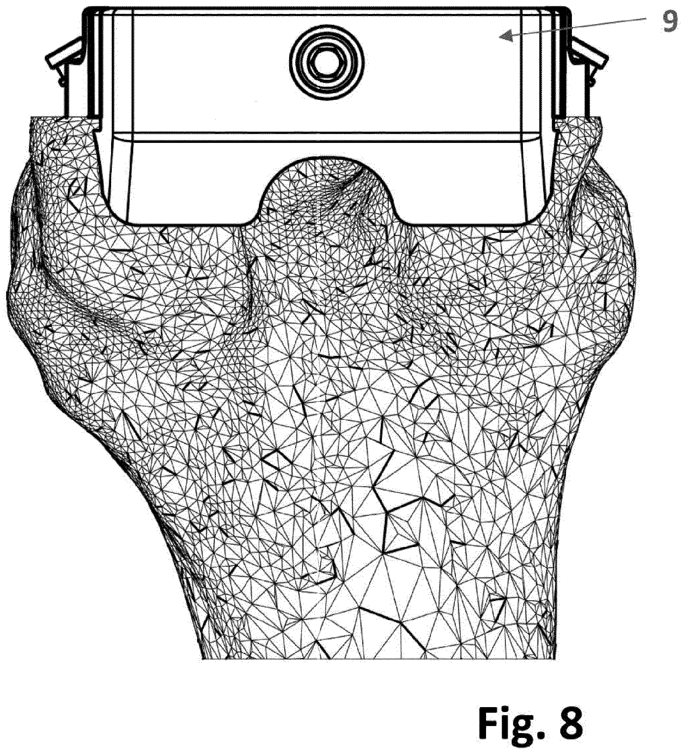

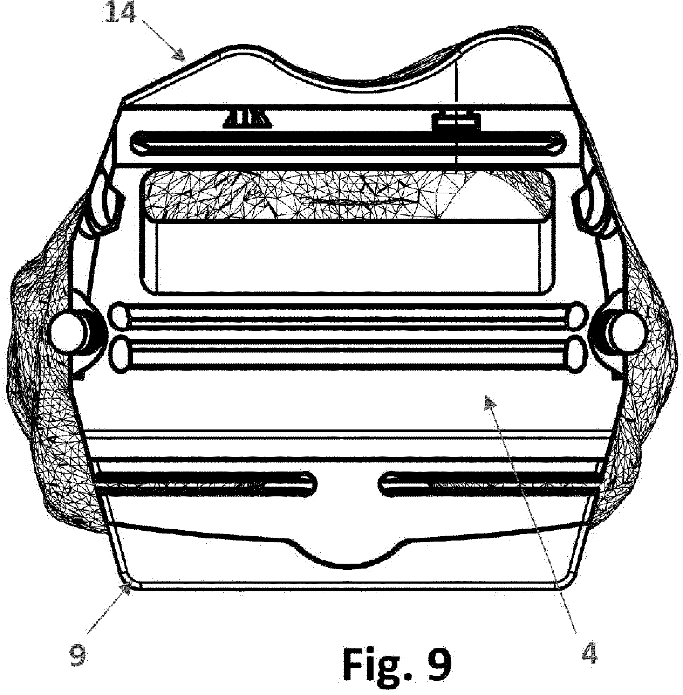

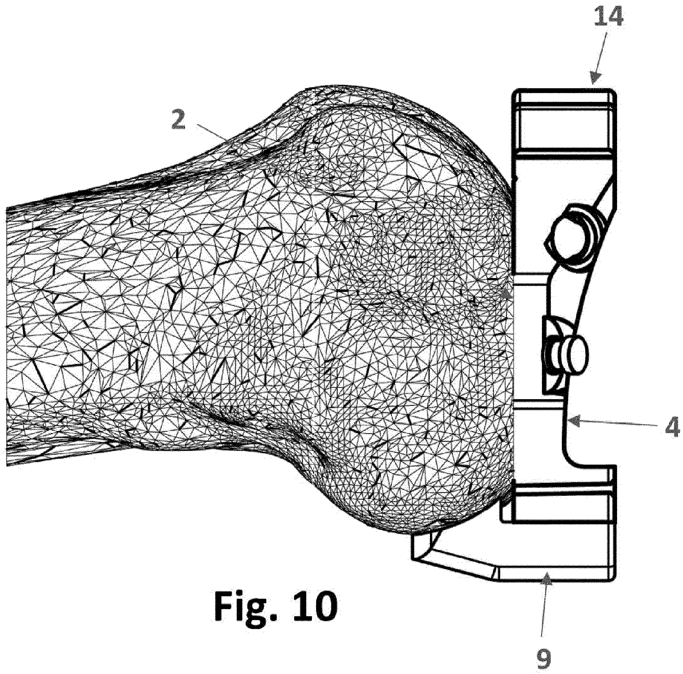

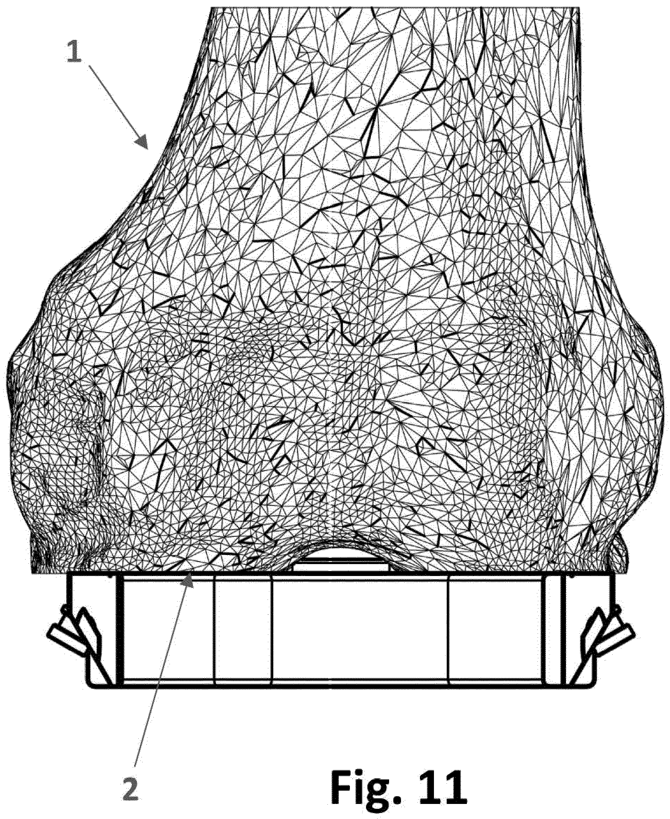

The first reference means, in a first embodiment illustrated in figures 1 - 11, consist of a first and respectively a second abutment element 11, 12 having a first surface 11a and respectively a second surface 12a configured to line up, during use, with the lateral femoral and respectively medial femoral condyle of the patient.

The first reference means, in another embodiment, consist of a single abutment element 13 having a wedge-shaped surface 13a configured to line up, during use, with the femoral intracondyloid fossa of the patient.

The first coupling means in an obligatory position 10 comprise geometrically conjugate surfaces 10a, 10b of the first module 9 and the cutting block 4.

The geometrically conjugate surfaces 10a, 10b can be curved surfaces having protuberances and recesses for a simple guided coupling.

The first coupling means in an obligatory position 10 comprise at least one locking screw 10c for locking between the geometrically conjugate surfaces 10a, 10b of the first module 9 and the cutting block 4.

Advantageously, there is also provided at least a second patient-specific made-to-measure module 14 and second coupling means 15 in an obligatory position for coupling the second module 14 with the cutting block 4.

The second module 14, in turn, has second reference means configured to position, during use, the cutting block 4 in a predetermined patient-specific manner relative to the distal resection surface 2.

The second reference means comprise a profile 16 of the second module 14 configured to be aligned, during use, with anterior anatomical landmarks of the femur adjacent to the distal resection surface 2.

The second coupling means in an obligatory position 15 comprise quick connect elements between the second module 14 and the cutting block 4.

The quick connect elements can comprise a pin 18 having an enlarged head 19 and an attachment member 20 projecting from a flat surface 21 of the second module 14 which mates with a flat front surface 22 of the cutting block 4.

The pin 18 engages along a guide groove 23 of the flat front surface 22 of the cutting block 4.

The guide groove 23 apically has a perimeter lip which interferes with the enlarged head 19 of the pin 18, preventing the extraction of the pin 18 from the guide groove 23 in the axial direction of the pin 18.

The attachment member 20 engages along an arcuate guide groove 25 of the flat front surface 22 of the cutting block 4.

The fastening member 20 and the guide groove 25 have mating tapered cross sections.

The arc defined by the guide groove 25 is centred on the pin 18.

The guide groove 25 has, in an intermediate position of the length thereof, two opposing recesses 25a where corresponding flexible tabs 26 provided on the attachment member 20 are engaged.

In practice, in order to fasten the second module 14 to the cutting block 4, one introduces and slides the pin 18 along the guide groove 23 all the way to the end.

Then one rotates the second module 14 around the pin 18 until inserting and sliding the attachment member 20 along the guide groove 25.

The rotation of the second module 14 concludes when the flexible tabs 26 snap fit into the recesses 25a.

The cutting jig 3 is adapted to the morphology of the femur of the specific patient, in particular it is adapted on the basis of morphological data relating to specific landmarks on which it will be applied in order to connect to the femur in a unique manner.

During an investigation phase of the preoperative study, images of the femur are acquired in order to recreate a three-dimensional morphology of the femur itself, for example by means of images obtained by computed tomography.

Subsequently, on the basis of the information derived during the investigation phase, the first module 9 and the second module 14 are made to measure for the specific patient and then fastened to the cutting block 4 to create the cutting jig 3. As mentioned, the cutting block 4 has four guides 5, 6, 7, and 8, each of which is configured to guide, during use, a cutting instrument for the execution of the anterior and posterior resections and the anterior and posterior chamfers, and in particular: a front guide 8 for the anterior resection, a front guide 7 for the anterior chamfer, a rear guide 6 for the posterior chamfer and a rear guide 5 for the posterior resection.

The cutting guides 5, 6, 7 and 8 are rectilinear through guides, that is to say, they extend through the entire thickness of the cutting block 4.

The due cutting guides 5 and 8 extend orthogonally and the other two cutting guides 6 and 7 extend with an inclination relative to the two parallel flat surfaces, proximal and distal, of the cutting block 4.

Advantageously, the cutting jig 3 is made of a material suitable for being used in contact with organic tissues; for example, it is made, at least partly, of plastic material, in particular of polymeric material such as, for example, polyamide.

In particular, the cutting block 4 can be made of steel and the modules 9, 14 of plastic material.

The modules 9, 14 of the jig 3 can be produced by means of a 3D printing system, for example by means of a laser sintering process.

The cutting block 4 of the jig 3 further has a plurality of holes 29, each of which is a through hole and configured to guide pins 30 configured to fasten, in a known manner, the jig 3 to the distal resection surface 2 of the femur.

When the jig 3 is applied on the distal resection surface 1, the abutment elements 10, 11, 12 of the jig 3 are disposed in contact with the predetermined landmarks of the femur.

It follows from what has been disclosed above that the cutting jig of the above-disclosed type will drastically reduce, if not eliminate, the possibilities of error during the operating phase due to an incorrect positioning of the cutting jig relative to the distal resection surface or an incorrect adjustment of the inclination of the guides to impart an internal or external rotation to the articulation of the prosthesis.

Furthermore, use of the jig of the above-described type is fast and simple, since the femoral condyles and the intracondyloid fossa represent easily identifiable landmarks.

The customisation of the jig of the above-described type based on the morphology of the patient's femur enables and facilitates, moreover, the insertion and positioning of the jig itself.

Finally, the jig of the above-described type may be stably fastened to the distal resection surface and enables the unique positioning to be maintained throughout the period of the operation.

## Claims

1. A 4-in-1 cutting jig for femoral finishing comprising a cutting block (4) provided with cutting guides (5, 6, 7, 8) configured to be placed, during use, in contact with the distal resection surface of a femur, wherein it comprises at least a first patient-specific made-to-measure module (9) and first coupling means (10) in an obligatory position for coupling said first module (9) with said cutting block (4), said first module (9) having first reference means configured to position, during use, said cutting block (4) in a predetermined patient-specific manner relative to said distal resection surface, said first reference means comprising at least a first abutment element (11, 12, 13) having a first surface configured to line up, during use, with a femoral landmark of the patient, wherein said cutting block (4) is a reusable universal cutting block (4) whilst said first module (9) is replaceable and renders the cutting jig patient specific, said cutting block (4) and said first module (9) being made of a different material, said first module (9) being produced with additive technology, wherein said first coupling means in an obligatory position comprise geometrically conjugate surfaces (10a, 10b) of said first module (9) and said cutting block (4), wherein said first coupling means in an obligatory position comprise at least one locking screw (10c) for locking between said geometrically conjugate surfaces (10a, 10b) of said first module (9) and said cutting block (4), _and wherein said 4-in-1 cutting jig comprises at least a second patient-specific made-to-measure module (14) and second coupling means (15) in an obligatory position for coupling said second module (14) with said cutting block (4), said second module (14) having second reference means configured to position, during use, said cutting block (4) in a predetermined patient-specific manner relative to said distal resection surface, said second reference means comprising a profile of said second module configured to be aligned, during use, with anterior anatomical landmarks of the femur.

2. The 4-in-1 cutting jig for femoral finishing according to claim 1, **characterised in that** said first reference means consist of a first and respectively a second abutment element (11, 12) having a first and respectively a second surface (11a, 12a) configured to line up, during use, with the lateral femoral and respectively medial femoral condyle of the patient.

3. The 4-in-1 cutting jig for femoral finishing according to claim 1, **characterised in that** said first reference means consist of a single abutment element (13) having a wedge-shaped surface (13a) configured to line up, during use, with the femoral intracondyloid fossa of the patient.

4. The 4-in-1 cutting jig for femoral finishing according to one of the preceding claims, **characterised in that** said second coupling means in an obligatory position comprise quick connect elements (15) for connecting between said second module (14) and said cutting block (4).

5. The 4-in-1 cutting jig for femoral finishing according to one of the preceding claims, **characterised in that** said cutting block (4) has, distributed along a first axis parallel to the anteroposterior axis of the femur:
- a front guide (8) having a first guide surface configured to guide, during use, a cutting instrument during the execution of an anterior resection;
- a front guide (7) having a second guide surface configured to guide, during use, a cutting instrument during the execution of an anterior chamfer;
- a rear guide (6) having a third guide surface configured to guide, during use, a cutting instrument during the execution of a posterior chamfer;
- a rear guide (5) having a fourth guide surface configured to guide, during use, a cutting instrument during the execution of a posterior resection.

6. The jig according to one of the preceding claims, **characterised in that** said second module (14) is produced with additive technology.

## Patentansprüche

1. Eine 4-in-1-Schneidlehre zur Femur-Endbearbeitung, umfassend einen Schneidblock (4), der mit Schneidführungen (5, 6, 7, 8) versehen ist, die so ausgelegt sind, dass sie während des Gebrauchs in Kontakt mit der distalen Resektionsfläche eines Femurs gebracht werden, wobei sie mindestens ein erstes patientenspezifisches, maßgefertigtes Modul (9) und erste Kopplungsmittel (10) in einer Zwangsposition umfasst, um das erste Modul (9) mit dem Schneidblock (4) zu koppeln, wobei das erste Modul (9) erste Bezugsmittel aufweist, die so konfiguriert sind, dass sie während des Gebrauchs den Schneidblock (4) in einer vorbestimmten, patientenspezifischen Weise relativ zu der distalen Resektionsfläche positionieren, wobei die ersten Bezugsmittel mindestens ein erstes Anlageelement (11, 12, 13) umfassen, das eine erste Oberfläche aufweist, die so ausgelegt ist, dass sie während des Gebrauchs mit einem femoralen Referenzpunkt des Patienten fluchtet, wobei der Schneidblock (4) ein wiederverwendbarer universeller Schneidblock (4) ist, während das erste Modul (9) austauschbar ist und die Schneidlehre patientenspezifisch macht, wobei der Schneidblock (4) und das erste Modul (9) aus unterschiedlichen Materialien bestehen, wobei das erste Modul (9) mittels additiver Fertigung hergestellt ist, wobei die ersten Kopplungsmittel in Zwangsposition geometrisch komplementäre Flächen (10a, 10b) des ersten Moduls (9) und des Schneidblocks (4) umfassen, wobei die ersten Kopplungsmittel in Zwangsposition mindestens eine Verriegelungsschraube (10c) zum Verriegeln zwischen den geometrisch komplementären Flächen (10a, 10b) des ersten Moduls (9) und des Schneidblocks (4) umfassen, und wobei die 4-in-1-Schneidlehre mindestens ein zweites patientenspezifisches, maßgefertigtes Modul (14) und zweite Kopplungsmittel (15) in Zwangsposition zum Koppeln des zweiten Moduls (14) mit dem Schneidblock (4) umfasst, wobei das zweite Modul (14) zweite Bezugsmittel aufweist, die so konfiguriert sind, dass sie während des Gebrauchs den Schneidblock (4) in einer vorbestimmten patientenspezifischen Weise relativ zu der distalen Resektionsfläche positionieren, wobei die zweiten Bezugsmittel ein Profil des zweiten Moduls umfassen, das so ausgelegt ist, dass es während des Gebrauchs mit anterioren anatomischen Landmarken des Femurs fluchtet.

2. Die 4-in-1-Schneidlehre nach Anspruch 1, **dadurch gekennzeichnet, dass** die ersten Bezugsmittel aus einem ersten und einem zweiten Anlageelement (11, 12) bestehen, die jeweils eine erste bzw. zweite Oberfläche (11a, 12a) aufweisen, die so ausgelegt sind, dass sie während des Gebrauchs mit dem lateralen bzw. medialen Femurkondylus des Patienten fluchten.

3. Die 4-in-1-Schneidlehre nach Anspruch 1, **dadurch gekennzeichnet, dass** die ersten Bezugsmittel aus einem einzigen Anlageelement (13) bestehen, das eine keilförmige Oberfläche (13a) aufweist, die so ausgelegt ist, dass sie während des Gebrauchs mit der femoralen Fossa intercondylaris des Patienten fluchtet.

4. Die 4-in-1-Schneidlehre nach einem der vorhergehenden Ansprüche, **dadurch gekennzeichnet, dass** die zweiten Kopplungsmittel in Zwangsposition Schnellverbindungselemente (15) zum Verbinden des zweiten Moduls (14) mit dem Schneidblock (4) umfassen.

5. Die 4-in-1-Schneidlehre nach einem der vorhergehenden Ansprüche, **dadurch gekennzeichnet, dass** der Schneidblock (4) entlang einer ersten Achse, die parallel zur anteroposterioren Achse des Femurs verläuft, verteilt ist und umfasst:
- eine vordere Führung (8) mit einer ersten Führungsfläche, die so konfiguriert ist, dass sie während des Gebrauchs ein Schneidinstrument bei der Durchführung einer anterioren Resektion führt;
- eine vordere Führung (7) mit einer zweiten Führungsfläche, die so konfiguriert ist, dass sie während des Gebrauchs ein Schneidinstrument bei der Durchführung einer anterioren Fase führt;
- eine hintere Führung (6) mit einer dritten Führungsfläche, die so konfiguriert ist, dass sie während des Gebrauchs ein Schneidinstrument bei der Durchführung einer posterioren Fase führt;
- eine hintere Führung (5) mit einer vierten Führungsfläche, die so konfiguriert ist, dass sie während des Gebrauchs ein Schneidinstrument bei der Durchführung einer posterioren Resektion führt.

6. Die Schneidlehre nach einem der vorhergehenden Ansprüche, **dadurch gekennzeichnet, dass** das zweite Modul (14) mittels additiver Fertigung hergestellt ist.

## Revendications

1. Gabarit de coupe 4-en-1 pour la finition fémorale, comprenant un bloc de coupe (4) muni de guides de coupe (5, 6, 7, 8) conçus pour être placés, lors de l'utilisation, en contact avec la surface de résection distale d'un fémur, lequel comprend au moins un premier module sur mesure spécifique au patient (9) et des premiers moyens d'accouplement (10) en position obligatoire pour l'accouplement dudit premier module (9) avec ledit bloc de coupe (4), ledit premier module (9) comportant des premiers moyens de référence configurés pour positionner, lors de l'utilisation, ledit bloc de coupe (4) de manière prédéterminée et spécifique au patient par rapport à ladite surface de résection distale, lesdits premiers moyens de référence comprenant au moins un premier élément d'appui (11, 12, 13) présentant une première surface conçue pour s'aligner, lors de l'utilisation, sur un repère fémoral du patient, le bloc de coupe (4) étant un bloc de coupe universel réutilisable, tandis que ledit premier module (9) est remplaçable et rend le gabarit de coupe spécifique au patient, ledit bloc de coupe (4) et ledit premier module (9) étant réalisés en matériaux différents, ledit premier module (9) étant fabriqué par une technologie additive, lesdits premiers moyens d'accouplement en position obligatoire comprenant des surfaces géométriquement conjuguées (10a, 10b) dudit premier module (9) et dudit bloc de coupe (4), lesdits premiers moyens d'accouplement en position obligatoire comprenant au moins une vis de verrouillage (10c) destinée à verrouiller les surfaces géométriquement conjuguées (10a, 10b) du premier module (9) et du bloc de coupe (4), et ledit gabarit de coupe 4-en-1 comprenant au moins un second module sur mesure spécifique au patient (14) et des seconds moyens d'accouplement (15) en position obligatoire pour l'accouplement dudit second module (14) avec ledit bloc de coupe (4), ledit second module (14) comportant des seconds moyens de référence configurés pour positionner, lors de l'utilisation, ledit bloc de coupe (4) de manière prédéterminée et spécifique au patient par rapport à ladite surface de résection distale, lesdits seconds moyens de référence comprenant un profil dudit second module conçu pour être aligné, lors de l'utilisation, sur des repères anatomiques antérieurs du fémur.

2. Gabarit de coupe 4-en-1 pour la finition fémorale selon la revendication 1, **caractérisé en ce que** lesdits premiers moyens de référence sont constitués d'un premier et d'un second élément d'appui (11, 12) présentant respectivement une première et une seconde surface (11a, 12a) conçues pour s'aligner, lors de l'utilisation, sur le condyle fémoral latéral et respectivement sur le condyle fémoral médial du patient.

3. Gabarit de coupe 4-en-1 pour la finition fémorale selon la revendication 1, **caractérisé en ce que** lesdits premiers moyens de référence sont constitués d'un seul élément d'appui (13) présentant une surface en forme de coin (13a) conçue pour s'aligner, lors de l'utilisation, sur la fosse intercondylaire fémorale du patient.

4. Gabarit de coupe 4-en-1 pour la finition fémorale selon l'une quelconque des revendications précédentes, **caractérisé en ce que** lesdits seconds moyens d'accouplement en position obligatoire comprennent des éléments de connexion rapide (15) pour le raccordement entre ledit second module (14) et ledit bloc de coupe (4).

5. Gabarit de coupe 4-en-1 pour la finition fémorale selon l'une quelconque des revendications précédentes, **caractérisé en ce que** ledit bloc de coupe (4) présente, répartis le long d'un premier axe parallèle à l'axe antéro-postérieur du fémur :
- un guide avant (8) présentant une première surface de guidage configurée pour guider, lors de l'utilisation, un instrument de coupe lors de l'exécution d'une résection antérieure ;
- un guide avant (7) présentant une seconde surface de guidage configurée pour guider, lors de l'utilisation, un instrument de coupe lors de l'exécution d'un chanfrein antérieur ;
- un guide arrière (6) présentant une troisième surface de guidage configurée pour guider, lors de l'utilisation, un instrument de coupe lors de l'exécution d'un chanfrein postérieur ;
- un guide arrière (5) présentant une quatrième surface de guidage configurée pour guider, lors de l'utilisation, un instrument de coupe lors de l'exécution d'une résection postérieure.

6. Gabarit selon l'une quelconque des revendications précédentes, **caractérisé en ce que** ledit second module (14) est fabriqué par une technologie additive.
